# EUROPÄISCHE PATENTSCHRIFT

(11) **EP 0 409 039 B1**
(45) Veröffentlichungstag und Bekanntmachung des Hinweises auf die Patenterteilung: **02.11.1994**
(21) Anmeldenummer: 90113095.5
(22) Anmeldetag: 09.07.1990
(51) Int. Cl.: C12M 1/02, C12M 1/04, C12M 3/02

(54) **Bioreaktor**
Bioreactor
Bioreacteur

(30) Priorität: 19.07.1989 DE 3923928
(43) Veröffentlichungstag der Anmeldung: 23.01.1991
(73) Patentinhaber: Stahl, Werner, Dr., D-76829 Landau (DE)
(72) Erfinder: Stahl, Werner, Dr., D-76829 Landau (DE)
(74) Vertreter: Weber, Otto Ernst, Dipl.-Phys.

(56) Entgegenhaltungen:
- CH-A- 295 385
- DE-A- 2 440 079
- FR-A- 1 604 693
- FR-A- 2 289 094
- FR-A- 2 353 638
- NL-A- 8 203 579
- US-A- 2 962 268
- US-A- 3 998 435
- US-A- 4 204 774

## Beschreibung

Die Erfindung betrifft einen Bioreaktor mit einem Reaktorgefäß mit mindestens einer Öffnung, die von mindestens einer Abdeckung verschlossen ist, mit mindestens einem durch die Abdeckung hindurchgeführten Rührwerkzeug, insbesondere in der Art eines venezianischen Rührers, dessen Lagerung an dem von der Durchführung nach außen weisenden Teil des Rührwerkzeugs angeordnet ist, wobei das Rührwerkzeug an seinem mit der Durchführung nach außen weisenden Teil mit einem Rührantrieb verbunden ist.

Ein gattungsgemäßer Bioreaktor ist aus der NL-A 8 203 579 bekannt. Dieser bekannte Bioreaktor ist zwar zum schonenden und gleichmäßigen Rühren einer Substanz geeignet, jedoch besteht keine geeignete Möglichkeit einer Anreichung der Substanz mit einem Fluid.

Aus der FR-A 1 604 693 ist ein Kippreaktor bekannt, bei dem über ein Rührorgan Luft einbringbar ist. An den Luftaustrittsöffnungen des Rührorgans entstehen jedoch aufgrund des Einpreßdrucks Verwirbelungen mit starken Scherbeanspruchungen, welche jedoch auf Biomassen, insbesondere Saugetierzellen, einen negativen Einfluß haben.

Besonders bei Bioreaktoren ist es wichtig, die Rührenergie möglichst gleichmäßig in das Volumen des Reaktorgefäßes einzubringen, um hohe Scherspannungen zu vermeiden. Gerade bei Rührern, die mit drehenden Wellen betrieben werden, z.B. bei Turbinenblattrührern, treten jedoch an den Kanten der Rührorgane sehr hohe Schergradienten auf, die zur Zerstörung von empfindlichen organischen Zellen führen können.

Aus dem deutschen Gebrauchsmuster 74 35 369 ist eine Rühranordnung mit einem Rührbehälter und einer Abdeckung bekannt, wobei die Abdeckung einen elastischen Bereich aufweist, an dem ein Rührstab gelagert ist. Der Rührstab wird durch einen Antrieb in eine Rührbewegung versetzt. Diese Anordnung ist jedoch für den Labormaßstab gedacht und in einer großtechnischen Ausführung nicht realisierbar, da die Trageeigenschaften und auch die elastischen Eigenschaften des elastischen Bereichs der Abdeckung bei einer größeren Dimensionierung stark verringert bzw. vermindert werden.

Der Erfindung liegt die **Aufgabe** zugrunde, einen Bioreaktor der gattungsgemäßen Art zu schaffen, der eine schonende und gleichmäßige Fluidzuführung in eine in dem Bioreaktor befindliche Substanz erlaubt und der auch im großtechnischen Maßstab einsetzbar ist.

Diese Aufgabe wird bei einem Bioreaktor der gattungsgemäßen Art erfindungsgemäß dadurch gelöst, daß das Rührwerkzeug einen mit einer Diffusionsschicht umgebenen Käfig mit einer Fluidzuführung aufweist.

Das Rührwerkzeug kann in einer vorteilhaften Weise zur Anreicherung, insbesondere Sauerstoffanreicherung der im Reaktorgefäß befindlichen Masse verwendet werden, wenn das Rührwerkzeug einen mit einer Diffusionsschicht umgebenen Käfig mit einer Fluidzuführung aufweist. Die Diffusionsschicht ist hierbei vorzugsweise als Gewebe, insbesondere als Edelmetallgewebe, ausgebildet. Der Sauerstoff wird hierdurch der im Reaktorgefäß befindlichen Masse oder Substanz gleichmäßig und schonend zugeführt und durch die Rührbewegung des Rührwerkzeugs werden Unterschiede der Sauerstoffkonzentration im Volumen des Reaktorgefäßes verringert. Der Sauerstoffeintrag auf diese Weise kann als indirekte Sauerstoffeintragung bezeichnet werden, die vor allem für Massen mit empfindlichen Zellen, z.B. Säugetierzellen, geeignet ist.

Es wird auch erreicht, daß die Funktion der Lagerung des Rührelements und der Abdichtung des Recktorgefäßes aufgeteilt werden. Auf diese Weise kann auch bei einer großtechnischen Realisierung eines Bioreaktors eine dünnere elastische Dichtung verwendet werden, die den Anforderungen an die Elastizität der Dichtung bei der Auslenkung des Rührwerkzeugs gerecht wird. Die Dichtung dient hierbei nicht zur Lagerung des Rührwerkzeugs. Die Lagerung des Rührwerkzeugs ist vorzugsweise als kardanische Aufhängung ausgebildet und über der Dichtung angeordnet, wodurch die Lagerung auf einfache Weise gewartet werden kann. Das Rührwerkzeug ist dabei in der Art eines venezianischen Rührers oder eines Paddels ausgebildet, wobei Kanten des Rührwerkzeugs abgerundet sind, um das Einbringen hoher Scherkräfte in die zu durchrührende Substanz zu vermeiden. Das Rührwerkzeug kann beispielsweise als Rührstab ausgebildet sein, der das Profil eines C hat. Der Strömungswiderstand ist dann abhängig von der Bewegungsrichtung des Rührstabs, da der Strömungswiderstand des Rührstabs zu verschiedenen Seiten hin unterschiedlich ist.

Das Rührwerkzeug bzw. der Rührstab kann in verschiedenen Arten bewegt werden. Zweckmäßig sind kreis- oder ellipsenförmige Bewegungen. Als sehr vorteilhaft erweisen sich hingegen taumelartige Bewegungen, die man sich als Überlagerung zweier kreisförmiger Bewegungen vorstellen kann.

Das Rührwerkzeug kann beispielsweise auch in der Art eines Pendels an seinem oberen Ende an einem Kreuzkopfgelenk aufgehängt sein, wobei die Auslenkung des Rührwerkzeugs durch einen etwas weiter unten angreifenden Schubkurbelantrieb erfolgen kann. In einer vorteilhaften Weiterbildung der Erfindung ist jedoch die Lagerung für das Rührwerkzeug im Bereich der Durchführung angeordnet. Der Rührantrieb kann dann beispielsweise ein Kurvenscheibengetriebe haben, das am oberen Ende des Rührwerkzeugs angreift.

In einer vorteilhaften Weiterbildung der Erfindung hat der Rührantrieb zur Erzeugung einer Taumelbewegung des Rührwerkzeugs eine angetriebene Kreisscheibe, an der azentrisch eine weitere, ebenfalls drehbar angetriebene zweite Kreisscheibe befestigt ist, in der azentrisch eine Führung für das Rührwerkzeug ausgebildet ist. Die Taumelbewegung entspricht in etwa der manuellen Rührbewegung beim Durchrühren chemischer oder biologischer Proben. Vor allem zum Durchrühren biologischer Substanzen mit empfindlichen Zellkonturen ist diese Art von Rührbewegung vorteilhaft, weil die Rührwirkung gleichmäßig unter Vermeidung hoher Scherkräfte auf die Substanz übertragen wird.

Im Gegensatz zu neueren Rührverfahren mit drehenden Wellen, wie z.B. Turbinenradrührer, entspricht das Rühren mit einer Taumelbewegung eher einem alten Rührprinzip, was hinsichtlich der oben beschriebenen Anwendungsfälle vorteilhaft sein kann.

Um ein Mitrotieren der in dem Reaktorgefäß befindlichen Flüssigkeit zu verhindern, können in dem Reaktorgefäß Stromstörer ausgebildet sein. Vorzugsweise haben die Stromstörer die Form von Kanten, die von der Innenwand des Reaktorgefäßes zur Gefäßmitte weisen. Zur Vermeidung einer sich einstellenden laminaren Strömung kann die Drehrichtung des Rührwerkzeugs auch periodisch umgekehrt werden, wobei zwischen den Drehrichtungswechseln Ruhepausen eingelegt werden können, bei denen die Trägheit der flüssigen Masse ausgenutzt wird, um Verwirbelungen an dem stillstehenden Rührwerkzeug zu erzeugen. In diesem Fall wirkt das Rührwerkzeug selbst als Stromstörer.

Bei dem oben beschriebenen Arbeitsverfahren erübrigt sich eventuell das Vorsehen von Stromstörern, wodurch ein kostengünstigeres Reaktorgefäß verwendet werden kann. Ein Reaktorgefäß ohne Stromstörer ist auch hinsichtlich der Wartungs- und Reinigungsarbeiten gegenüber Reaktorgefäßen mit Stromstörern vorteilhaft.

Bei toxischen Produkten möchte man aus Sicherheitsgründen eine Mehrfachsperre in Form hintereinander geschalteter Dichtungen vorsehen. Daher ist in einer vorteilhaften Ausbildung der Erfindung ein elastischer Bereich der Abdeckung durch zwei zueinander parallel angeordnete gummielastischen Dichtungen gebildet. Diese gummielastischen Dichtungen sind zwischen dem Rührwerkzeug und einem festen Teil der Abdeckung ausgebildet, wobei der Zwischenraum zwischen den Dichtungen mit einer Sperrflüssigkeit gefüllt ist, die relativ zu dem Innenraum des Reaktorgefäßes mindestens unter gleichem Druck, insbesondere unter leichtem Überdruck steht. Bei einem Ausfall einer Dichtung bleiben immer noch zwei weitere Dichtungssperren wirksam. Der Druck der Sperrflüssigkeit in dem Zwischenraum kann durch eine Steuerung überwacht und über Zu- und Ableitungen eingestellt werden. Giftige Dämpfe entstehen beispielsweise beim anaeroben Abbau von biologischem Material.

In einer vorteilhaften Weiterbildung der Erfindung hat das Rührwerkzeug einen Rührstab, an dem mindestens eine Schaufelradanordnung in der Art eines Windmeßrades drehfest gelagert ist. Bei einer Rührbewegung des Rührstabes wird die Schaufelradanordnung ebenfalls mit ausgelenkt und erzielt damit ihrerseits einen Rühreffekt. In diesem Sinne ist es dann vorteilhaft, wenn die Schaufeln des Schaufelrades als querhalbierte Kreisellipsoide ausgebildet sind, deren Schnittfläche konkav gekrümmt ist. Die Schaufeln haben dann in beiden axialen Richtungen des Kreisellipsoids einen sehr unterschiedlichen Strömungswiderstand, der die Rührbewegung der Schaufelradanordnung verstärkt.

Statt der Schaufelradanordnungen können an dem Rührstab auch mehrere, in etwa parallel zu diesem angeordnete paddelartig ausgebildete Rührleisten an propellerartigen Aufhängungen drehfest gelagert sein. Diese Rührleisten haben vorzugsweise ein Profil, das dem des querhalbierten Kreisellipsoids entspricht. Auf diese Weise wird eine großflächige und gleichmäßige Rührwirkung erzielt. Wenn die Rührleisten zum Boden des Reaktorgefäßes leicht konisch auf den Rührstab zulaufen, liegen die Leisten in ihrer der Wand des Reaktorgefäßes zugewandten Stellung etwa parallel zur Wand des Reaktorgefäßes. Der Abstand zwischen diesen kann dann möglichst gering gehalten werden, wodurch eine gute Durchrührung des gesamten Volumens des Reaktorgefäßes erreicht wird.

Anstelle der querhalbierten Kreisellipsoide oder der Rührleisten können auch andere Rührkörper mit folgenden Eigenschaften für die am Rührstab befestigten Anordnungen verwendet werden. Die Rührkörper müssen einen großen Unterschied des Strömungswiderstands zwischen der Vor- und Rückwärtsbewegung und einen hohen Seitenwiderstand haben. Auf diese Weise wird die drehfest gelagerte Anordnung in eine schnelle Drehbewegung versetzt, wobei durch die große Lateralfläche eine starke Verwirbelung bei der Seitenbewegung erzielt wird. Als Betriebsparameter können die Drehzahl und die Exzentrizität des Taumelrührers verändert werden.

Das Rührwerkzeug kann in einer einfachen und kostengünstigen Ausbildung der Erfindung durch einen Exzenterantrieb in eine Rührbewegung versetzt werden.

In einer sehr betriebssicheren Ausführung wird das Rührwerkzeug durch einen Antrieb mit einem Planetengetriebe in eine Rührbewegung versetzt. Die Antriebswelle eines Motors trägt dabei ein Sonnenrad des Planetengetriebes, während eine kardanische Lagerung für das obere Teil des Rührwerkzeugs an dem Käfig für die Planetenräder angeordnet ist. Die Planetenräder sind zwischen dem Sonnenrad und einem innen verzahnten Ring gelagert. Wenn diese Planetenräder gleich groß sind, werden die Planetenräder bei einer Drehung des Sonnenrads in eine langsamere Drehung versetzt, die auf den Käfig und damit über die kardanische Lagerung auf das Rührwerkzeug übertragen wird. Mit einem derartigen Antrieb lassen sich sehr große Kräfte über eine lange Betriebszeit übertragen. Ein derartiger Antrieb eignet sich daher sehr gut als Antrieb für die großtechnische Ausführung eines Bioreaktors.

In einer vorteilhaften Weiterbildung der Erfindung trägt das Rührwerkzeug mindestens einen Schaumzerstörer, der eine Vielzahl von Wänden aufweist, die parallel zur Achse des Rührwerkzeugs ausgebildet sind. Durch einen derartigen Schaumzerstörer läßt sich zum einen eine gute Durchmischung der im Reaktorgefäß befindlichen Masse erreichen und zum anderen wird die Ausbildung einer störenden Schaumkrone verhindert. Derartige Schaumkronen wirken sich hinsichtlich konstanter Arbeitsbedingungen sehr störend aus.

Eine gleichmäßige und über ein großes Volumen verteilte Durchrührung der im Reaktorgefäß befindlichen Masse wird erreicht, wenn mehrere Rührwerkzeuge an verschiedenen Seiten des Reaktorgefäßes angeordnet sind.

Vorteilhafte Weiterbildungen sind Gegenstand der Unteransprüche.

Die Erfindung wird nachfolgend beispielsweise anhand der schematischen Zeichnung beschrieben. In dieser zeigen:
- Fig. 1: eine Seitenansicht eines Bioreaktors mit einem als paddelförmiger Rührstab ausgebildeten Rührwerkzeug;
- Fig. 2: eine detaillierte Seitenansicht einer Durchführung eines Rührwerkzeugs durch eine Abdeckung;
- Fig. 3: eine Seitenansicht eines Rührstabs mit einer an dessen unterem Ende angeordneten Schaufelradanordnung;
- Fig. 4: die Aufsicht IV-IV aus Fig. 3;
- Fig. 5: einen Bioreaktor, der einen Rührstab mit drei Schaufelradanordnungen hat;
- Fig. 6: einen Querschnitt durch ein Planetengetriebe als Antrieb für ein Rührwerkzeug;
- Fig. 7: eine teilgeschnittene Seitenansicht eines Bioreaktors mit einem als Schaumzerstörer ausgebildeten Rührwerkzeug;
- Fig. 8: einen Bioreaktor mit mehreren synchron angetriebenen Rührwerkzeugen;
- Fig. 9: einen Querschnitt eines Rührwerkzeugs zum Sauerstoffeintrag in die im Reaktorraum befindliche Masse; und
- Fig. 10: den Querschnitt eines Bioreaktors mit Strömungszerstörern.

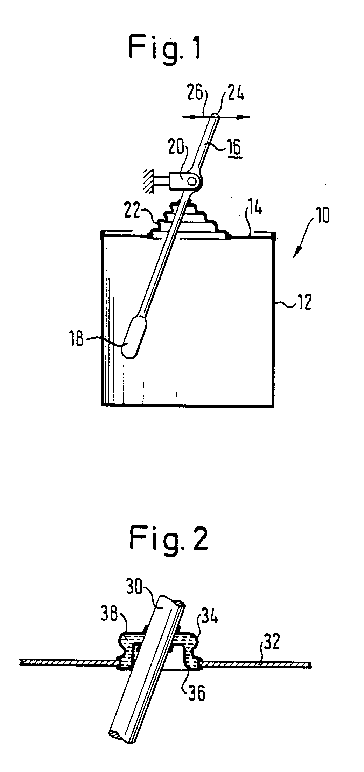

Der in Fig. 1 gezeigte Bioreaktor 10 hat ein kreiszylindrisches Reaktorgefäß 12, dessen offene Oberseite von einer Abdeckung 14 verschlossen ist. Die Abdeckung 14 hat eine zentrale Aussparung, durch die ein als Rührstab ausgebildetes Rührwerkzeug 16 in den Reaktorraum im Innern des Reaktorgefäßes 12 hinein geführt ist. Der Rührstab 16 ist an seinem unteren Ende 18 paddelförmig verbreitert. Über der Durchführung des Rührstabs 16 durch die Abdeckung 14 ist der Rührstab 16 an einer kardanischen Aufhängung 20 gelagert.Im Bereich der Durchführung ist der Rührstab 16 über eine elastische Gummimuffe 22 mit der Abdeckung 14 gasdicht verbunden. Das obere Ende 24 des Rührstabs 16 wird von einem nur schematisch angedeuteten Antrieb 26 parallel zur Ebene der Abdeckung 14 ausgelenkt. Auf diese Weise kann der Rührstab 16 in beliebiger Weise geschwenkt werden. Hierbei sind kreisförmige oder elliptische Bahnen vorstellbar. Vorzugsweise wird der Rührstab 16 jedoch in taumelnder Weise bewegt. Man kann sich die Taumelbewegung als zwei überlagerte azentrische Kreisbewegungen vorstellen.

In Fig. 2 ist der Bereich einer Durchführung eines Rührstabs 30 durch eine Abdeckung 32 ähnlich dem in Fig. 1 beschriebenen Beispiel dargestellt. Der Rührstab 30 und die Abdeckung 32 sind durch zwei im wesentlichen parallen zueinander angeordnete gummielastische Dichtungen 34,36 miteinander verbunden. Der Zwischenraum 38 zwischen den beiden Dichtungen 34 und 36 ist mit einer Sperrflüssigkeit gefüllt, die relativ zum Reaktorraum unter einem leichten Überdruck steht. Auf diese Weise wird auch bei Beschädigung einer Dichtung noch eine sichere Dichtwirkung aufrechterhalten. Es ist hierbei darauf zu achten, daß die Sperrflüssigkeit nicht toxisch auf die im Reaktorraum befindliche Substanz wirkt. Der Druck der Sperrflüssigkeit kann über nicht dargestellte Sensoren konstant überwacht werden und über nicht dargestellte Zu- und Ableitungen reguliert werden.

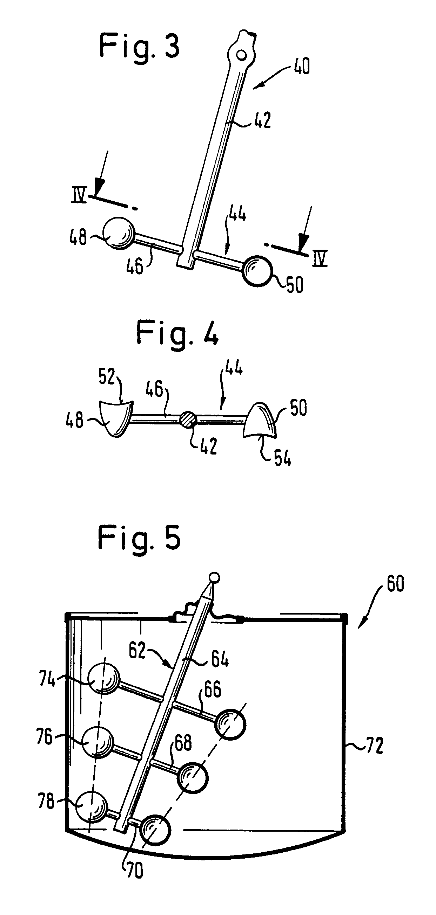

In Fig. 3 ist ein Rührwerkzeug 40 dargestellt, das im Prinzip anstelle des Rührstabs 16 in dem Bioreaktor 10 aus Fig. 1 verwendet werden kann. Das Rührwerkzeug 40 besteht aus einem Rührstab 42, an dessen unterem Ende eine Schaufelradanordnung 44 in der Art eines Windmeßrades drehfest gelagert ist.

In Fig. 4 ist die Aufsicht auf die Schaufelradanordnung 44 in axialer Richtung des Rührstabes 42 aufgetragen. Die Schaufelradanordnung 44 besteht aus einer propellerartigen Verbindungsstrebe 46, die in der Mitte an dem Rührstab 42 drehfest gelagert ist. An den beiden äußeren Enden der propellerartigen Verbindungsstrebe 46 ist jeweils ein querhalbiertes Kreisellipsoid 48,50 angeordnet, wobei die Schnittfläche 52,54 der Kreisellipsoide konkav gekrümmt ist.

Fig. 5 zeigt einen Bioreaktor 60, dessen Rührwerkzeug 62 aus einem Rührstab 64 mit drei Schaufelradanordnungen 66,68,70 besteht. Die Länge der Verbindungsstreben der drei Schaufelradanordnungen 66,68,70 nimmt von der oberen Schaufelradanordnung 66 zur unteren Schaufelradanordnung 70 hin ab. Hierdurch ist bei allen drei Schaufelradanordnungen 66,68,70 der Abstand der quergeschnittenen Kreisellipsoide 74,76,78 in ihrer der Reaktorwand 72 zugewandten Stellung im wesentlichen gleich. Die drei quergeschnittenen Kreisellipsoide 74,76,78 liegen somit auf einer zum unteren Ende des Rührstabs 64 spitz zulaufenden,konischen Kegelfläche, deren Wände gestrichelt angedeutet sind. Die drehfest gelagerten Schaufelradanordnungen 66,68,70 können jeweils zwei, drei oder mehr Rührelemente in Form der quergeschnittenen Kreisellipsoide aufweisen.

Mit Bezug auf Fig. 4 kann es empfehlenswert sein, den Übergang von der Mantelfläche der quergeschnittenen Kreisellipsoide 48, 50 zu deren Schnittfläche 52,54 abzurunden, um Zellen, die sich in der im Reaktorgefäß aufzubereitenden biologischen Substanz befinden, nicht zu zerstören. Durch die Ausbildung der Rührelemente als quergeschnittene Kreisellipsoide treten in der zu verrührenden Substanz oder Flüssigkeit nur geringere Scherkräfte auf, die ein Bearbeiten auch empfindlicher biologischer Substanz erlauben.

Hinsichtlich der in Fig. 5 beschriebenen Ausführungsform ist es weiterhin möglich, die Kreisellipsoide 74,76,78 durch eine Rührleiste zu ersetzen, die gemäß der gestrichelten Linie ausgerichtet ist. Die Rührleiste hat dann einen Querschnitt, der dem Längsschnitt des quergeschnittenen Kreisellipsoids 48 oder 50 (Fig. 4) entspricht.

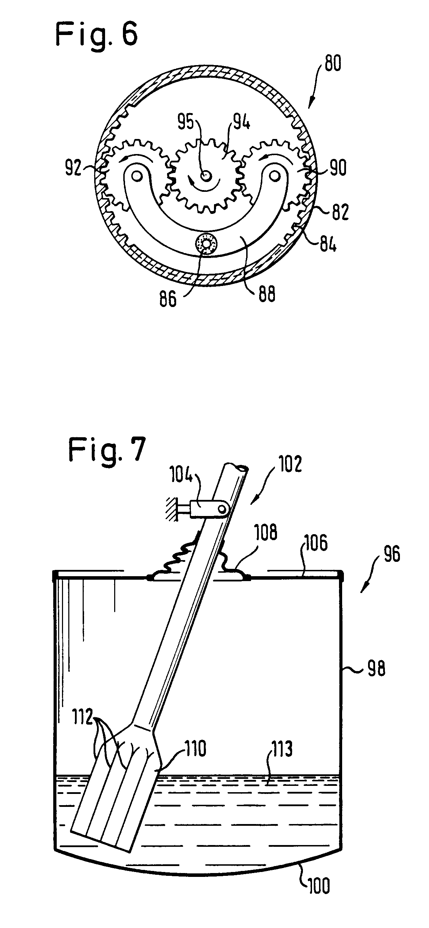

In Fig. 6 ist die teilgeschnittene Aufsicht auf ein Planetengetriebe 80 zum Antrieb eines Rührwerkzeugs dargestellt. Das Planetengetriebe 80 besteht aus einer ringförmigen Außenwand 82 mit einer Innenverzahnung 84, aus zwei durch einen Käfig 88 verbundenen Planetenrädern 90,92 und einem zwischen diesen angeordneten Sonnenrad 94, welches auf der Welle 95 eines Motors befestigt ist. Die beiden Planetenräder 90 und 92 haben den gleichen Durchmesser und die gleiche Anzahl an Zähnen. An dem Käfig 88 zum Verbinden der beiden Planetenräder ist eine Kugelgelenkführung 86 für das obere Teil des Rührwerkzeugs ausgebildet. Der Antrieb 80 ist über dem Reaktorgefäß angeordnet, so daß bei einer Drehung der Welle 95 die Kugelgelenkführung 86 für das Rührwerkzeug eine Kreisbewegung ausführt. Durch die Kugelgelenkführung 86 kann das schwenkende Oberteil des Rührwerkzeugs mit dem sich drehenden Käfig 88 des Planetengetriebes verbunden werden. Durch diesen Antrieb 80 können sehr große Kräfte über lange Zeit auf das Rührwerkzeug übertragen werden.

Fig. 7 zeigt einen weiteren Bioreaktor 96 mit einem kreiszylindrischen Reaktorgefäß 98, dessen Boden 100 konvex nach unten gewölbt ist, damit das untere Ende eines Rührwerkzeugs über den gesamten Schwenkbereich einen im wesentlichen gleichen Abstand vom Boden des Reaktorgefäßes hat. Der Bioreaktor 96 hat ein Rührwerkzeug 102, das an seinem oberen Ende in nicht dargestellter Weise durch einen Antrieb ausgelenkt wird. Das Rührwerkzeug 102 ist an einer kardanischen Aufhängung 104 gelagert. Zwischen einer Abdeckung 106 des Reaktorgefäßes und dem Rührwerkzeug 102 ist eine Gummimuffe 108 in der Art eines Wellenrohrkompensators zur Abdichtung vorgesehen. Das Rührwerkzeug 102 hat an seinem unteren Ende einen Schaumzerstörer 110, der mehrere konzentrische kreiszylindrische oder nach oben hin konzentrisch zulaufende (nicht dargestellte)Rohre aufweist. An den Wänden 112 dieser Rohre schlägt sich der Schaum nieder und tropft bei einer stärkeren, insbesondere kreisförmigen Bewegung des Schaumzerstörers 110 wieder in die im Reaktorgefäß 98 befindliche Flüssigkeit 114 ab.

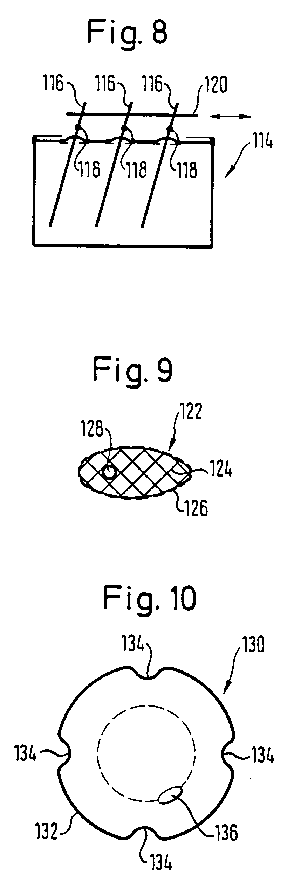

In Fig. 8 ist stark schematisch ein Bioreaktor 114 mit drei in Reihe angeordneten Rührwerkzeugen 116 dargestellt. Die Rührwerkzeuge sind an Aufhängepunkten 118 gelagert. An ihrem oberen Ende sind die Rührwerkzeuge durch eine Schub- und Zugstange 120 mit einem nicht dargestellten Antrieb verbunden. Die Rührwerkzeuge 116 werden auf diese Weise synchron ausgelenkt.

In Fig. 9 ist der Querschnitt eines Rührstabs 122 dargestellt. Mit diesem in der Art eines paddelförmigen Rührstabs ausgebildeten Rührwerkzeug 122 lassen sich Fluide, vor allem Sauerstoff, in die zu rührende Substanz oder Flüssigkeit einbringen. Der Rührstab besteht aus einem Käfig 124, der von einem sehr feinmaschigen Edelstahlgeflecht 126 umgeben ist. Diese Art der Sauerstoffzuführung kann als indirekter Sauerstoffeintrag in eine zu verrührende organische Substanz bezeichnet werden, wobei diese Art des Sauerstoffeintrags für sehr empfindliche Zellen, wie z.B. Säugetierzellen, vorgesehen ist. Das Fluid bzw. der Sauerstoff wird dem Käfig über eine Zuführung 128 zugeführt. Die Sauerstoffblasen koagulieren an der Edelstahlgewebe-Oberfläche 126 und treten nicht durch diese hindurch. Der Sauerstoff wird in der Flüssigkeit gelöst durch Diffusion und/oder Flüssigkeitstransport durch die Maschen des Gewebes 126 in den mit wachsenden Zellen gefüllten Reaktorraum. Durch die Bewegung des Rührwerkzeugs in Diffusionsrichtung wird die Diffusion bzw. der Flüssigkeitstransport beschleunigt. Gleichzeitig werden durch die Rührwirkung des Rührwerkzeugs 122 Konzentrationsunterschiede des Sauerstoffs in der im Reaktorraum befindlichen Flüssigkeit vermieden. Durch eine Taumelbewegung beim Rühren wird sowohl eine gute Rührwirkung als auch eine schnelle Diffusion des Sauerstoffs in die Flüssigkeit erzielt. Es können statt eines Rührwerkzeugs 122 ein ganzer Satz derartiger Rührwerkzeuge, z.B. in der in Fig. 8 beschriebenen Weise betätigt werden. Derartige Käfige können weiterhin auch als Schaufelradanordnung gemäß den Fig. 3 bis 5 ausgebildet sein, wobei dann dafür zu sorgen ist, daß an den Übergangsstellen vom Rührstab zu den Schaufelradanordnungen eine Sauerstoffzuführung ermöglicht wird. Derartige Käfige können auch kreiszylindrisch ausgebildet sein.

In Fig. 10 ist der Querschnitt eines Bioreaktors 130 dargestellt. Das kreiszylindrische Reaktorgefäß 132 hat in Umfangsrichtung um 90° versetzt vier in den Reaktorinnenraum hineinragende Leisten 134, die als Stromstörer dienen. Auf diese Weise wird auch bei einer kreisförmigen Rührbewegung eines Rührwerkzeugs 136 eine ausreichende Verwirbelung der im Reaktorgefäß 132 befindlichen Masse bewirkt.

Selbstverständlich ist es möglich, Pakete von vorstehend genannten Rührwerkzeugen, z.B. zehn Stück, synchron zu bewegen. Es ist weiterhin möglich, Rührwerkzeuge von mehreren verschiedenen Seiten, z.B. auch von den Seitenwänden aus, in das Reaktorgefäß hineinzuführen, wodurch dann eine gleichmäßigere Durchrührung der im Reaktorgefäß befindlichen Flüssigkeit erreicht wird, da zur Erzielung einer gleichen Rührwirkung der Ausschlag jedes einzelnen Rührwerkzeugs geringer sein kann als wenn nur ein einziges Rührwerkzeug verwendet wird.

Durch die vorstehend genannten Ausführungsformen wird eine im Vergleich zu Propellerrührern oder Magnetstabrührern sehr schonende Rührwirkung erzielt, da die in die zu verrührende Masse eingebrachten Scherkräfte nicht so groß sind wie bei den bekannten Rührtechniken. Die beschriebenen Rührtechniken lassen sich insbesondere in Anlagen für den großtechnischen- oder Industriebedarf realisieren.

## Patentansprüche

1. Bioreaktor
mit einem Reaktorgefäß mit mindestens einer Öffnung, die von mindestens einer Abdeckung (14) verschlossen ist, mit mindestens einem durch die Abdeckung (14) hindurchgeführten Rührwerkzeug (16, 122), insbesondere in der Art eines venezianischen Rührers, dessen Lagerung (20) an dem von der Durchführung nach außen weisenden Teil des Rührwerkzeugs (16, 122) angeordnet ist, wobei das Rührwerkzeug (16,122) an seinem von der Durchführung nach außen weisenden Teil (24) mit einem Rührantrieb (26) verbunden ist,
dadurch **gekennzeichnet**,
daß das Rührwerkzeug (122) einen mit einer Diffusionsschicht (126) umgebenen Käfig (124) mit einer Fluidzuführung (128) aufweist.

2. Bioreaktor nach Anspruch 1,
dadurch **gekennzeichnet**,
daß die Diffusionsschicht (126) als Gewebe, insbesondere Edelmetallgewebe, ausgebildet ist.

3. Bioreaktor nach Anspruch 1 oder 2,
dadurch **gekennzeichnet**,
daß alle Teile des Rührwerkzeugs aus Hohlprofilen bestehen, die untereinander in Verbindung stehen, und daß schaufel-, paddel- oder leistenähnliche Rühranordnungen des Rührwerkzeugs als entsprechend geformte Käfige aus einem Edelmetallgeflecht ausgebildet sind, wobei den Käfigen durch den durch die Durchführung nach außen geführten Teil des Rührwerkzeugs Fluide zur gleichmäßigen Verteilung in dem Reaktorgefäß zuführbar sind.

4. Bioreaktor nach einem der Ansprüche 1 bis 3,
dadurch **gekennzeichnet**,
daß das Fluid Sauerstoff enthält.

5. Bioreaktor nach Anspruch 1,
dadurch **gekennzeichnet**,
daß der Rührantrieb ein Kurvenscheibengetriebe hat.

6. Bioreaktor nach Anspruch 1,
dadurch **gekennzeichnet**,
daß der Rührantrieb zur Erzeugung einer Taumelbewegung des Rührwerkzeugs eine angetriebene Kreisscheibe aufweist, an der azentrisch eine weitere ebenfalls drehbar angetriebene zweite Kreisscheibe befestigt ist, in der azentrisch eine Führung für das Rührwerkzeug ausgebildet ist.

7. Bioreaktor nach einem der vorhergehenden Ansprüche,
dadurch **gekennzeichnet**,
daß die Lagerung für das Rührwerkzeug im Bereich der Durchführung angeordnet ist.

8. Bioreaktor nach einem der vorhergehenden Ansprüche,
dadurch **gekennzeichnet**,
daß in dem Reaktorgefäß (132) Stromstörer (134) ausgebildet sind.

9. Bioreaktor nach Anspruch 8,
dadurch **gekennzeichnet**,
daß die Stromstörer (134) als von der Innenwand des Reaktorgefäßes (132) zur Gefäßmitte weisende Kanten ausgebildet sind.

10. Bioreaktor nach einem der vorhergehenden Ansprüche,
dadurch **gekennzeichnet**,
daß das Rührwerkzeug an seinem oberen Ende gelagert und weiter unten von einem Schubkurbelantrieb betätigt ist.

11. Bioreaktor nach einem der vorhergehenden Ansprüche,
dadurch **gekennzeichnet**,
daß ein elastischer Bereich der Abdeckung (32) durch zwei zueinander parallel angeordnete gummielastische Dichtungen (34,36) gebildet ist.

12. Bioreaktor nach Anspruch 11,
dadurch **gekennzeichnet**,
daß die gummielastischen Dichtungen (34,36) zwischen dem Rührwerkzeug (30) und einem festen Teil der Abdeckung (32) ausgebildet sind, und daß der Zwischenraum (38) zwischen den Dichtungen (34,36) mit einer Sperrflüssigkeit gefüllt ist, die relativ zum Innenraum des Reaktorgefäßes mindestens unter gleichem Druck, insbesondere unter leichtem Überdruck steht.

13. Bioreaktor nach einem der vorhergehenden Ansprüche,
dadurch **gekennzeichnet**,
daß das Rührwerkzeug als paddelförmiger Rührstab (18) ausgebildet ist.

14. Bioreaktor nach Anspruch 13,
dadurch **gekennzeichnet**,
daß der Rührstab eine asymmetrische Querschnittsfläche aufweist.

15. Bioreaktor nach einem der Ansprüche 1 bis 12,
dadurch **gekennzeichnet**,
daß das Rührwerkzeug (40) einen Rührstab (42) aufweist, an dem mindestens eine Schaufelradanordnung (44) in der Art eines Windmeßrades befestigt ist.

16. Bioreaktor nach Anspruch 15,
dadurch **gekennzeichnet**,
daß die Schaufeln (48,50) des Schaufelrads als querhalbierte Kreisellipsoide ausgebildet sind, deren Schnittfläche (52,54) konkav gekrümmt ist.

17. Bioreaktor nach einem der Ansprüche 1 bis 12,
dadurch **gekennzeichnet**,
daß das Rührwerkzeug mindestens einen Rührstab aufweist, an dem mehrere in etwa parallel zu diesem angeordnete paddelartig ausgebildete Rührleisten an propellerartigen Aufhängungen befestigt sind.

18. Bioreaktor nach Anspruch 17,
dadurch **gekennzeichnet**,
daß die Rührleisten zum Boden des Reaktorgefäßes leicht konisch auf den Rührstab zulaufen.

19. Bioreaktor nach einem der vorhergehenden Ansprüche,
dadurch **gekennzeichnet**,
daß der Antrieb des Rührwerkzeugs durch ein Planetengetriebe (80) gebildet ist.

20. Bioreaktor nach Anspruch 19,
dadurch **gekennzeichnet**,
daß an einem Käfig (88) für die Planetenräder (90,92) des Planetengetriebes eine Kugelgelenkführung (86) für das Rührwerkzeug ausgebildet ist.

21. Bioreaktor nach einem der vorhergehenden Ansprüche,
dadurch **gekennzeichnet**,
daß das Rührwerkzeug (102) mindestens einen Schaumzerstörer (110) trägt, der eine Vielzahl von Wänden (112) aufweist, die parallel zur Achse des Rührwerkzeugs (102) ausgebildet sind.

22. Bioreaktor nach Anspruch 21,
dadurch **gekennzeichnet**,
daß die Wände (112) als konzentrische Zylinder ausgebildet sind.

23. Bioreaktor nach einem der vorhergehenden Ansprüche,
dadurch **gekennzeichnet**,
daß mehrere Rührwerkzeuge an verschiedenen Seiten des Reaktorgefäßes angeordnet sind.

24. Bioreaktor nach Anspruch 1,
dadurch **gekennzeichnet**,
daß die Lagerung für das Rührwerkzeug durch den Antrieb gebildet ist.

25. Bioreaktor nach Anspruch 24,
dadurch **gekennzeichnet**,
daß das Rührwerkzeug durch den Rührantrieb in eine Translationsbewegung versetzbar ist.

## Claims

1. A bioreactor with a reactor vessel with at least one opening, which is closed by at least one cover (14), with at least one stirring apparatus (16,122) to be passed through the cover (14), particularly in the manner of a Venezian stirrer, wherein a mounting (20) for the stirring apparatus (16, 122) is provided on the part of the stirring apparatus (16,122) directed outwards from the passage, whilst at its part (24) directed outwards from the passage the stirring apparatus (16,122) is connected to a stirring drive (26),
wherein the stirring apparatus (122) has a cage (124) with a fluid supply (128) surrounded by a diffusion layer (126).

2. A bioreactor according to claim 1,
wherein the diffusion layer (126) is constructed as a gauze, particularly a precious metal gauze.

3. A bioreactor according to claim 1 or 2,
wherein all parts of the stirring apparatus comprise hollow profiles, which are interconnected and wherein blade, paddle or strip-like stirring means of the stirring apparatus are constructed as correspondingly shaped precious metal gauze cages, which can be supplied with fluids for uniform distribution in the reactor vessel through the stirring apparatus part led outwards through the passage.

4. A bioreactor according to one of the claims 1 to 3,
wherein the fluid contains oxygen.

5. A bioreactor according to claim 1,
wherein the stirring drive has a disk cam mechanism.

6. A bioreactor according to claim 1,
wherein the stirring drive for producing a tumbling movement of the stirring apparatus has a driven circular disk, to which is acentrically fixed a further second circular disk also driven in rotary manner and in which is acentrically constructed a guide for the stirring apparatus.

7. A bioreactor according to one of the preceding claims,
wherein the mounting for the stirring apparatus is located in the vicinity of the passage.

8. A bioreactor according to one of the preceding claims,
wherein flow disturbers (134) are constructed in the reactor vessel (132).

9. A bioreactor according to claim 8,
wherein the flow disturbers (134) are constructed as edges directed from the inner wall of the reactor vessel (132) towards the centre thereof.

10. A bioreactor according to one of the preceding claims,
wherein the stirring apparatus is mounted at its upper end and is operated further downwards by a slider crank drive.

11. A bioreactor according to one of the preceding claims,
wherein an elastic area of the cover (32) is formed by two parallel rubber-elastic seals (34,36).

12. A bioreactor according to claim 11,
wherein the rubber-elastic seals (34,36) are constructed between the stirring apparatus (30) and a fixed part of the cover (32) and wherein the gap (38) between the seals (34,36) is filled with a sealing liquid, which is at least under the same pressure and in particular is under a slight overpressure relative to the inner area of the reactor vessel.

13. A bioreactor according to one of the preceding claims,
wherein the stirring apparatus is constructed as a paddle-like stirring rod (18).

14. A bioreactor according to claim 13,
wherein the stirring rod has an asymmetrical cross-sectional surface.

15. A bioreactor according to one of the claims 1 to 12,
wherein the stirring apparatus (40) has a stirring rod (42), to which is fixed at least one impeller arrangement (44) in the manner of a wind measuring wheel.

16. A bioreactor according to claim 15,
wherein the blades (48,50) of the impeller are constructed as transversely bisected ellipsoids of revolution, whose sectional plane (52,54) has a concave curvature.

17. A bioreactor according to one of the claims 1 to 12,
wherein the stirring apparatus has at least one stirring rod, on which several paddle-like stirring strips on propeller-like suspensions are fixed roughly parallel thereto.

18. A bioreactor according to claim 17,
wherein the stirring strips towards the bottom of the reactor vessel taper slightly conically towards the stirring rod.

19. A bioreactor according to one of the preceding claims,
wherein the stirring apparatus drive is formed by a planetary gear (80).

20. A bioreactor according to claim 19,
wherein a spherical joint guide (86) for the stirring apparatus is constructed on a cage (88) for the planetary wheels (90,92) of the planetary gear.

21. A bioreactor according to one of the preceding claims,
wherein the stirring apparatus (102) carries at least one foam breaker (110), which has a plurality of walls (112) constructed parallel to the stirring apparatus axis (102).

22. A bioreactor according to claim 21,
wherein the walls (112) are constructed as concentric cylinders.

23. A bioreactor according to one of the preceding claims,
wherein several stirring apparatuses are provided on different sides of the reactor vessel.

24. A bioreactor according to claim 1,
wherein the mounting for the stirring apparatus is formed by the drive.

25. A bioreactor according to claim 24,
wherein the stirring apparatus can be given a translatory movement by the stirring drive.

## Revendications

1. Bioréacteur comportant une cuve de réacteur avec au moins une ouverture qui est fermée par au moins un couvercle (14), au moins un dispositif agitateur (16, 122) qui traverse le couvercle (14), notamment un dispositif agitateur du type vénitien dont le palier (20) est disposé sur la partie du dispositif agitateur (16, 122) qui est tournée vers l'extérieur par rapport au passage, le dispositif agitateur (16, 122) au niveau de sa partie (24) tournée vers l'extérieur par rapport au passage étant lié à un mécanisme d'entraînement (26), caractérisé par le fait que le dispositif agitateur (122) comporte une cage (124) qui est entourée d'une couche de diffusion (126) et présente une arrivée de fluide (128).

2. Bioréacteur selon la revendication 1, caractérisé par le fait que la couche de diffusion (126) est agencée sous forme de tissus, en particulier sous forme de tissus de métal précieux.

3. Bioréacteur selon la revendication 1 ou 2, caractérisé par le fait que toutes les parties du dispositif agitateur sont constituées par des profilés creux qui communiquent entre eux et par le fait que des systèmes d'agitation de type augets, palettes ou tiges du dispositif agitateur sont agencés sous la forme de cages adaptées en tissu de métal précieux des fluides pouvant être envoyés aux cages à travers la partie du dispositif agitateur amenée à l'extérieur par l'intermédiaire du passage aux fins de répartition homogène dans la cuve de réacteur.

4. Bioréacteur selon l'une des revendications 1 à 3, caractérisé par le fait que le fluide contient de l'oxygène.

5. Bioréacteur selon la revendication 1, caractérisé par le fait que le mécanisme d'entraînement de l'agitateur comporte un mécanisme à came.

6. Bioréacteur selon la revendication 1, caractérisé par le fait qu'afin de générer un mouvement de nutation du dispositif agitateur, le mécanisme d'entraînement comporte un plateau circulaire entraîné sur lequel est fixé de manière excentrée un deuxième plateau circulaire également entraîné en rotation dans lequel est aménagée une glissière excentrée pour le dispositif agitateur.

7. Bioréacteur selon l'une des revendications précédentes, caractérisé par le fait que le palier pour le dispositif agitateur est disposé dans la région du passage.

8. Bioréacteur selon l'une des revendications précédentes, caractérisé par le fait que des brise-lames (134) sont disposés à l'intérieur de la cuve de réacteur (132).

9. Bioréacteur selon la revendication 8, caractérisé par le fait que les brise-lames (134) sont agencés sous la forme de bords qui s'étendent depuis la paroi intérieure de la cuve de réacteur (132) en direction de l'intérieur de celle-ci.

10. Bioréacteur selon l'une des revendications précédentes, caractérisé par le fait que le dispositif agitateur est supporté au niveau de son extrémité supérieure et est entraîné plus loin vers le bas par un mécanisme à manivelle.

11. Bioréacteur selon l'une des revendications précédentes, caractérisé par le fait qu'une zone élastique du couvercle (32) est constituée par deux joints (34, 36) qui présentent l'élasticité du caoutchouc et sont disposés parallèlement l'un à l'autre.

12. Bioréacteur selon la revendication 11, caractérisé par le fait que les joints (34, 36) présentant l'élasticité du caoutchouc sont disposés entre le dispositif agitateur (30) et un partie fixe du couvercle (32) et par le fait que l'espace (38) entre les joints (34, 36) est rempli d'un liquide de barrage qui, par rapport à la chambre intérieure de la cuve de réacteur, est au moins à la même pression, notamment est à une pression légèrement supérieure.

13. Bioréacteur selon l'une des revendications précédentes, caractérisé par le fait que le dispositif agitateur est agencé sous forme de tige d'agitateur à palette (18).

14. Bioréacteur selon la revendication 13, caractérisé par le fait que la tige d' agitateur présente une section transversale asymétrique.

15. Bioréacteur selon l'une des revendications 1 à 12, caractérisé par le fait que le dispositif agitateur (40) comporte une tige d'agitateur (42) sur laquelle est fixé au moins un ensemble de roue à augets (44) semblable à une roue d'anémomètre.

16. Bioréacteur selon la revendication 15, caractérisé par le fait que les augets (48, 50) de la roue à augets ont la forme d'une ellipsoïde de révolution coupée en deux dans la direction transversale, dont la surface (52, 54) est concave.

17. Bioréacteur selon l'une des revendications 1 à 12, caractérisé par le fait que le dispositif agitateur comporte au moins une tige d'agitateur sur laquelle plusieurs barrettes d'agitateur en forme de palettes disposées sensiblement parallèlement à la tige sont fixées sur des supports en forme d'hélice.

18. Bioréacteur selon la revendication 17, caractérisé par le fait que les barrettes d'agitateur en direction du fond de la cuve de réacteur se rapprochent de la tige en formant un cône à faible pente.

19. Bioréacteur selon l'une des revendication précédentes, caractérisé par le fait que le mécanisme d'entraînement du dispositif agitateur est constitué par un engrenage planétaire (80).

20. Bioréacteur selon la revendication 19, caractérisé par le fait qu'un moyen de guidage à rotule (86) pour le dispositif agitateur est aménagé sur un carter (88) pour les roues planétaires (90, 92) de léngrenage planétaire.

21. Bioréacteur selon l'une des revendications précédentes, caractérisé par le fait que le dispositif agitateur (102) porte au moins un dispositif (110) de destruction de la mousse qui présente une pluralité de parois (112) disposées parallèlement à l'axe du dispositif agitateur (102).

22. Bioréacteur selon la revendication 21, caractérisé par le fait que les parois (112) sont agencées sous la forme de cylindre concentriques.

23. Bioréacteur selon l'une des revendications précédentes, caractérisé par le fait que plusieurs dispositifs agitateurs sont disposés sur différentes faces de la cuve de réacteur.

24. Bioréacteur selon la revendication 1, caractérisé par le fait que le support pour le dispositif agitateur est constitué par le mécanisme d'entraînement.

25. Bioréacteur selon la revendication 24, caractérisé par le fait que le dispositif agitateur peut être entraîné en un mouvement de translation par le mécanisme d'entraînement.
